(19) Europäisches Patentamt
European Patent Office
Office européen des brevets

(11) Publication number : **0 594 523 A1**

(12) **EUROPEAN PATENT APPLICATION**

(21) Application number : **93500116.4**

(22) Date of filing : **30.07.93**

(51) Int. Cl.$^5$ : **C08G 65/32**, C08G 65/22, C07D 303/16, C06B 45/10

(30) Priority : **31.07.92 ES 9201623**

(43) Date of publication of application : **27.04.94 Bulletin 94/17**

(84) Designated Contracting States : **DE FR GB**

(71) Applicant : **UNION ESPANOLA DE EXPLOSIVOS S.A.**
**Claudio Coello 124**
**E-28006 Madrid (ES)**

(72) Inventor : **Ochoa Gomez, Jose Ramon**
**Embarcaciones 25**
**E-28760 Tres Cantos (Madrid) (ES)**
Inventor : **Blanco Gomez, Juan Jose**
**Avda. Maria Luisa 9, Urbanizacion Montellano**
**E-28490 Becerril De La Sierra (Madrid) (ES)**

(74) Representative : **Garcia Cabrerizo, Francisco**
**OFICINA GARCIA CABRERIZO S.L. Vitruvio 23**
**E-28006 Madrid (ES)**

(54) **A process for producing energetic polymers starting with hydroxy-terminated polyglycidil nitrate.**

(57) A process for producing energetic polymers starting with hydroxy-terminated polyglycidil nitrate. Novel hydroxy-terminated glycidil azide-glycidil nitrate polymers (GAP-PGN) of formula (I) are described, wherein n is a whole number between 8 and 84 and m is a whole number greater than zero and smaller than n, obtained by reaction between a polyglycidil nitrate (II) polymer with a metallic azide in an organic solvent and optionally in the presence of a phase transfer catalyst (PTC). When the substitution of nitrate groups by azide groups is equal to or more than 95%, the invention offers hydroxy-terminated GAP (VII) homopolymer. Also is described how glycidil nitrate (GN) (III) is obtained starting with glycidol and a chloride of sulfonic acid.

The GAP-PGN (I) copolymers and GAP (VII) copolymers are useful as energetic links in the formulation of propelling solids for rockets.

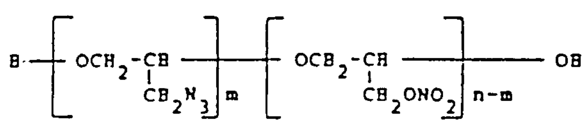

(I)

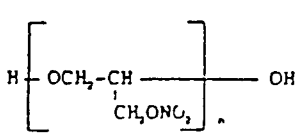

(II)

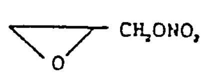

(III)

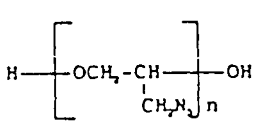

(VII)

EP 0 594 523 A1

## FIELD OF THE INVENTION

The invention relates to the synthesis of novel products, more particularly to homopolymers and hydroxy-terminated energetic copolymers of a molecular weight between 1,000 and 10,000 used in the synthesis of polyurethanes as energetic links in the formulation of solid propellants for rockets and satellites. More specifically, the invention relates partly to the synthesis of hydroxy-terminated azide-glycidil nitrate copolymers (GAP-PGN) heretofore not described, starting on the basis of polyglycidil nitrate (PGN) of molecular weight between 1000 and 10000 by reacting the same with a metallic azide in a polar organic solvent, optionally in the presence of a phase transfer catalyst (PTC).

On the other hand, in the specific case where the degree of substituting nitrate groups by azide groups is equal to or exceeds 95%, the invention refers to a novel process for synthesizing hydroxy-terminated polyglycidil azide (PGA) homopolymer.

The invention also relates to a novel process for obtaining glycidil nitrate (GN) a monomer necessary for producing PGN starting product for synthesizing the polymers of the present invention.

## PRIOR ART

Until this very day, hydroxy-terminated copolymers (GAP-PGN) have not been described. However, corresponding homopolymers have been described and thus, US patent 4486351, Australian A-60799/86, US 4268450 and Spanish patent application 9101804 claim various processes for producing GAP (azide glycidil polymers) starting with polyepichlorohydrin (PECH), while PCT application WO 90/15092 describes the synthesis of PGN (glycidil nitrate polymers). Given that both copolymers are endowed with characteristics which make them adequate for being used as energetic links in the formulation of solid propellants for rockets and satellites, it would be very desirable to join together in the same macromolecular chain the energetic groups of said homopolymers, since in such form could be controlled the interesting features of the same by simply designing their composition; the relative percentage of the azide and nitrate groups in the copolymers.

Consequently, the primary objective of the present invention is a process for synthesizing hydroxy-terminated GAP-PGN copolymers with functionality equal or exceeding 2, and the molecular weight being between 1000 and 10000, by means of a process which makes it possible to easily control their composition, consistent with the PGN reaction with the metallic azide in a polyorganic solvent, optionally in the presence of PTC. This process makes it possible to obtain GAP-PGN copolymers of any composition just by controlling the molar ratio of the metallic azide to PGN, the temperature and the period of reaction.

In a specific case where the degree of substitution is equal or higher than 95%, the obtained product is GAP. Presently, GAP is synthesized by the reaction of PECH with sodium azide in a polar organic solvent. Thus, in the obtained final product there remains residual chloride groups which do not contribute anything to its energetic power. Consequently, the second object of the invention is a process for producing GAP on the basis of a completely different raw material, the PGN, a polymer containing nitrate groups, also energetic, in its side chain. Thus, the obtained GAP on the basis of PGN will have residual groups in the case of keeping the same, nitrate groups which, in addition to contributing energy, do not obstruct the combustion as is the case ofchloride atoms whose retarding action of the flame is well known.

Although the synthesis of PGN is described, this product is not commercially available because each synthesis must be handled starting with a GN monomer which is commercially hardly available. The GN synthesis is described in various publications and patents. All of them have a common denominator: these are nitration reactions involving the risk that in the course of synthesis, by-products are obtained, as intermediate products or as explosive compounds. Thus, the synthesis described in Will.Ber. Deutsch. Chem Ges. 41, 1117 (1908) takes place through obtaining glycerol dinitrate cycled in a second stage; in J.Amer. Chem. Soc. 76 4385 (1954) the 3-chloropropane-1,2-diol is mononitrated and subsequently, cycled. The yield of both metals are low. L. T. Eremenko and A. M. Koroles Izvestiya Akademii Nauk SSSR. Seriya Khimischeskaya 5, 1142-1144 (1967) describe the synthesis of GN on the basis of glycidil by reacting with a mixture 16:26 of $NO_3H$ 100% and acetic anhydride at -10°C in 20 minutes. This is also a dangerous method because the mixture $NO_3H$/acetic anhydride internally generates acetyl nitrate, an unstable explosive product, and leads to producing a GN contaminated with significant amounts (2.5% p/p) dinitroacetates.

Recently, in patent applications PCT WO 90/01028 and WO 90/01029, the synthesis of GN on the basis of glycidil has been described by reaction with dinitrogenous pentoxides at temperatures between -10°C and -70°C in an inert organic solvent. This method produces high purity GN but contaminated with up to 0.5% nitroglycerin, a highly sensitive explosive product. Moreover, the process requires synthesizing $N_2O_5$, an unstable product difficult to handle.

On the other hand, in the glycidil reaction with $N_2O_5$, the intended product, (GN) and $NO_3H$, is generated;

this requires maintaining rigorous conditions of synthesis and of the processes for preventing $NO_3H$ from opening the cycle of glycidil nitrate. And so, the third object of the invention is to develop a secure process of synthesizing glycidil nitrate (GN) obviating the use of unstable and dangerous reactants as well as avoiding the possibility of the formation of explosive by-products.

## DETAILED DESCRIPTION OF THE INVENTION

1. <u>Synthesis of hydroxy-terminated GAP-PGN copolymers.</u>

The first object of the invention is a process for producing GAP-PGN copolymers of general formula (I)

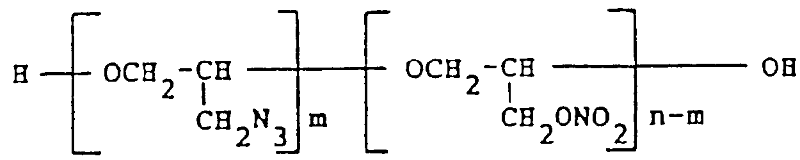

(I)

wherein n is a whole number between 8 and 84; and m is a whole number between 0 and less than n.

The hydroxy-terminated GAP-PGN copolymers of general formula (I) are produced by reacting hydroxy-terminated PGN of general formula (II) with a metallic azide in a polar organic solvent, at temperatures between 0 and 120°C, optionally in the presence of phase transfer catalyst (PTC) according to the following reaction plan:

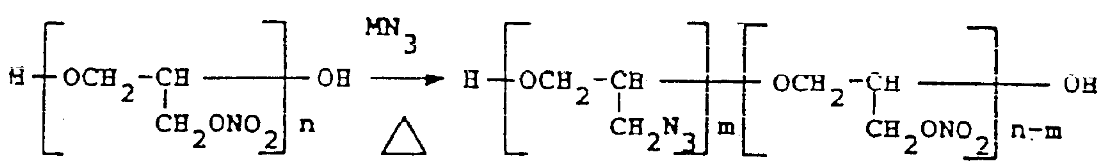


PGN (II) GAP-PGN (I)

wherein n is a whole number between 8 and 84; and m is a whole number greater than 0 and less than n.

The departure product PGN (II) is not the object of the present invention and can be obtained by a process described in the previously mentioned PCT WO 90/15092 patent.

The metallic azide ($MN_3$) which is used is generally sodium azide ($NaN_3$). The reaction is carried out in heterogeneous phase, the adequate solvents being polar organic solvents, particularly dimethyl acetamide (DMA), dimethyl formamide (DMF), N-methyl pyrolidon (NMP), N-methyl formamide (NMF), sulfolane, dimethyl sulfoxide (DMSO) and acetonitrile (ACN). The reaction can be accelerated by using PTC as quaternary ammonium salts, particularly p-toluene sulfonate of tetrabutyl ammonium and ammonium tetrabutyl nitrate. The bromides and chlorides of quaternary ammonium can be used but are not recommended because both the bromide as well as the chloride ion displaces the PGN nitrate group (II) and since the azide more easily displaces the nitrate group than the chloride or bromide, it becomes difficult to obtain GAP-PGN (I) copolymers of predetermined composition.

The PTC concentration can be ample but usually it suffices using between 1 and 15% molar relative to PGN nitrate groups (II).

The molar ratio of azide to PGN (II) depends on the intended copolymer composition and it is convenient to work with a slight excess stoichiometric for securing adequate substitution of nitrate groups by azide groups. Thus, said molar ratio of azide/PGN (II) can be between 0.2 and 0.3 when the intended composition is GAP-PGN 20/80; between 0.5 and 0.6 when it is 50/50, etc. Any other composition is produced by following the same criterion.

The concentration of PGN (II) in the medium of the reaction can be between 2 and 50% by weight (referring to soluble reactants; this is PGN and solvent), preferably between 20 and 40%.

The temperature can be between ambient temperature and 120°C, preferably between 50 and 100°C.

When the reaction is finished, the solvent is recovered by distillation either at atmospheric pressure or in vacuo. The residues of the same together with the inorganic salts and the PTC, if used, are extracted with water prior to the solution of the copolymer with dichloromethane (DCM). After the organic phase is decanted, drying follows with $Na_2SO_4$ or $MgSO_4$, filtering and DCM is recovered by distillation. The residue is GAP-PGN copolymer (I), as shown by the applied characterizing techniques: IR, $^1$H-NMR, $^{13}$C-NMR, elemental analysis and GPC (Gel Permeation Chromatography).

2. Synthesis of hydroxy-terminated GAP polyglycidil azide.

The second object of the invention is a process for producing GAP of a general formula (VII).

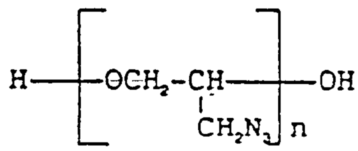 (VII)

According to the process of the invention, GAP (VII) is produced by reacting hydroxy-terminated PGN (II) with a metallic azide in a polar organic solvent at temperatures exceeding 50°C, in the presence of a phase transference catalyst (PTC) according to the following plan of reaction:

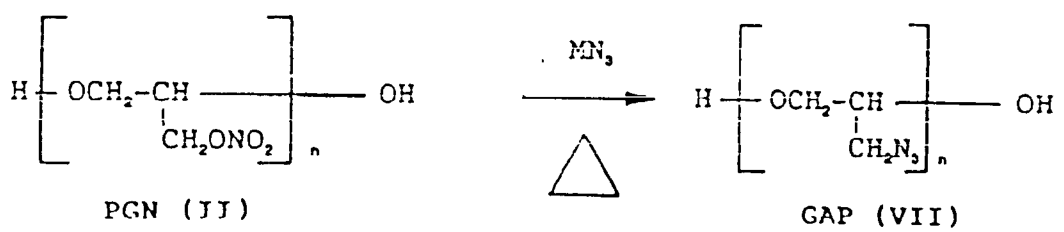


PGN (II) GAP (VII)

wherein n is a whole number between 8 and 84.

The metallic azide being used is generally sodium azide ($Na_2$). The reaction is carried out in heterogeneous phase since the azide is practically insoluble in organic solvents and the election of the solvent is primarily for achieving an adequate rate of reaction for high yield. The addition of quaternary ammonium salts as PTC accelerates the substitution, particularly p-toluene sulfonate of tetrabutyl ammonium and tetrabutyl ammonium nitrate.

The suitable solvents are: dimethyl acetamide (DMA), dimethyl formamide (DMF), dimethyl sulfoxide (DMSO), N-methyl pyrolidone (NMF), N-methyl formamide, sulfolane and acetonitryl (ACN), particularly DMA, DMF, DMSO and NMF.

The reaction temperature can be between 50 and 120°C, but in order that the period of reaction not be excessive, it is expedient if it is between 90 and 120°C.

The functionality of the departure PGN should be equal or higher than 2 and its molecular weight between 1200 (n=8) and 10000 (n=84). This product can be obtained by a process described in the patent application PCT WO 90/15092.

The PGN (II) concentration in the medium of the reaction can be between 2 and 50% by weight (referring to the soluble reactants, i.e. PGN and solvent), preferably between 20 and 30%.

The molar reaction of sodium azide to PGN (II) can be between 1 and 2, preferably 1.5.

The molar PTC (if used) relation to PGN (II) can be between 0.05 and 0.25, preferably between 0.1 and 0.15.

When the reaction is over, the solvent is recovered by distillation in vacuo. The residues of the same, together with $NaNO_3$ and $NaN_3$ without reacting, are extracted with water, with previous dissolution of GAP in dichloromethane (DCM). After the organic phase is decanted, drying follows with $Na_2SO_4$ or $MgSO_4$, filtering and DCM is recovered by distillation. The residue is GAP (VII), a viscous liquid whose color varies from amber to red.

3. Synthesis of glycidil nitrate (GN).

The second object of the invention is a process for synthesizing glycidil nitrate (GN) of the formula (III)

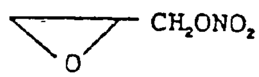

(III)

which is the monomer necessary for producing PGN (II).

According to the process of the present invention, the synthesis of GN (III) includes the following steps:

a) reaction of glycidol (IV)

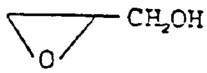

(IV)

with sulfonic chloride acid of the general formula (V)

R-$SO_2$-Cl (V)

wherein R can be a radical corresponding to any known sulfonic acid, preferably

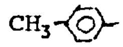

$CH_3$-, $CH_3$-$CH_2$ -, $CH_3$-$(CH_2)_2$-,
$CH_3$-$(CH_2)_3$ -;

at a temperature between ambient temperature and 50°C, in an inert organic solvent in the presence of an organic base such as pyridine or triethyl amine for producing a sulfonic ester of general formula (VI)

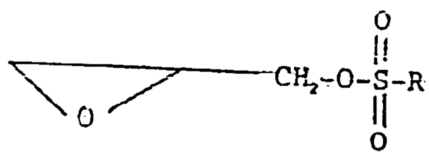

(VI)

wherein R can be of the previously quoted significance;

b) isolation of sulfonic ester (VI) resulting from the previous reaction and the recycle with the solvent being used;

c) reaction of sulfonic ester (VI) obtained in the previous stages with metallic nitrate in an organic solvent, particularly in the presence of a phase transfer catalyst (PTC), at temperatures between ambient temperature and 100°C, for arriving at glycidil nitrate (GN) of the previous general formula (III);

d) isolation of GN (III) by distillation in vacuo after recovery of the used solvent;

e) re-use of sulfonic ester (VI), without reacting, as well as of CTF if it is used.

The following reaction plan summarizes the process:

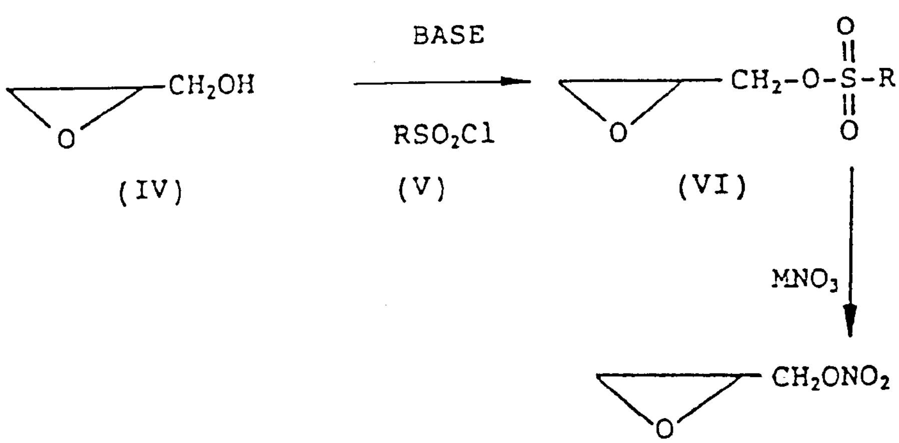


(IV) (V) (VI)

(III)

wherein R can be of the previously mentioned significance and
M is a metallic cation.

As a sulfonic chloride acid (V) can be used any known sulfonic chloride acid, preferably those where R is

$CH_3$ 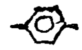 ,

$CH_3$-, $CH_3CH_2$-, $CH_3$-$(CH_2)_2$-,
$CH_3$-$(CH_2)_3$-.
However according to the present invention, the best results are obtained when R is a radical of toluene

( $CH_3$ 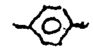 ),

i.e. when the sulfonic chloride acid being used is p-toluene sulfonile chloride.

As base can be used any organic base capable of rapidly reacting with chlorhydric acid generated in the reaction and, moreover, which does not interfere in the same. Particularly recommended are pyridine and triethylamine.

As solvent can be used any inert organic solvent. Particularly adopted are chlorated solvents, particularly carbon tetrachloride.

The synthesis of sulfonic ester (VI) can be carried out with pyridine as solvent acting at the same time as a base. However, in such cases, the results are not so satisfactory because polymers of low molecular weight are generated in a greater or lesser amount depending on the conditions of synthesis. They significantly obstruct the purification of the product and reduce the yield of the reaction. These problems are obviated by working with $CCl_4$ in the presence of a light excess of triethylamine with respect to the limiting reactant (sulfonic chloride acid (V)). For facilitating the isolation of the product (VI), it is expedient to operate with a molar excess of glycidil (IV) with relation to the acid chloride (V). Specifically, a molar excess between 5 and 30%.

The temperature can be between ambient temperature and 50°C and the concentration of glycidol (IV) between 1 and 20% p/p, preferably between 5 and 10%.

Under these conditions, a yield is obtained of sulfonic ester (VI) between 90 and 95% in a reaction period not exceeding 6 h. When the reaction is finished, the chlorhydrate of the organic base, which precipitates in the course of the same, is extracted with water. The organic phase is dried with $Na_2SO_4$ or $MgSO_4$, anhydride, and is filtered and the solvent is recovered by distillation. The last traces of the solvent are eliminated by distillation in vacuo and the residual is sulfonic ester (VI) of glycidil. The isolated output is equal to or more than 90% and the purity of the product exceeds 98%. It is of solid white or yellowish white from the fusion between 34 and 36°C in the case of p-toluene sulfonate of glycidil.

As already mentioned, the second part of the process consists in producing GN (III) by reacting sulfonic ester (VI) of glycidol with a metallic nitrate as indicated in the previous plan of reaction.

Any metal can be M, but the results are obtained when M is sodium. The reaction can be carried out in a plurality of solvents, such as DMA, NMF, NMP, sulfolane, DMSO, tetrahydrofuran (THF), dioxane, DMF, acetonitrile, dichloromethane, chloroform, carbon tetrachloride, 1,2-dichloroethane and others.

In the case of using solvents such as DMA, NMF, NMP, sulfolane, DMF and DMSO, it is not expedient to use a PTC. The reaction takes place without difficulty at temperatures exceeding 80°C. However, the separation of GN (III) with such types of solvents is not easy and entails losses in the yield and the formation of subproducts which contaminate GN (III) and, in some cases, interfere with or impede its polymerization. All of these problems can be obviated by using solvents of boiling points lower than 100°C in the presence of a PTC. For example, $CH_2Cl_3$, $CHCl_3$, $CCl_4$ and acetonitrile. As PTC can be used any quaternary ammonium salt whose anion has low nucleophilic power. Particularly suitable are p-toluene sulfonate of tetrabutyl ammonium and tetrabutyl ammonium nitrate.

According to the process of the present invention, surprising results are obtained by combining any of the 2 cited quaternary ammonium salts with acetonitrile as solvent since when the reaction is finished, the precipitate formed by sodium nitrate and sodium p-toluene sulfonate is separated by filtration, the acetonitrile is recovered by distillation in vacuo (60-100 mmHg) and the GN (III) is recovered with a high degree of purity of 99% by distillation in vacuo (1-3 mmHg) taking care that the temperature of the boiler should not exceed 100°C. The residual in the boiler is constituted by sulfonic ester of glycidil without reaction, GN without recovery and quaternary ammonium salt.

According to the process of the invention, such residual can be recirculated in a greater number of cycles. Thus, by only adding a new charge of sulfonic ester (VI) and sodium nitrate and acetonitrile recovered by distillation, another reaction is obtained without it being necessary to supply a new amount of quaternary ammonium salt.

The temperature can be between ambient temperature and that of the reflux of the solvent. An adequate temperature is between 80 and 85°C and then reaction does not last more than 5 h.

The concentration of sulfonic ester (VI) can be between 30 and 60% by weight. The molar ratio $NaNO_3$/sulfonic ester (VI) can be between 1 and 3 and the molar ratio of quaternary ammonium salt (PTC/sulfonic ester (VI) between 0.05 and 0.20, preferably between 0.1 and 0.15.

The yield of obtained GN (III) is on the order of 85-90% and the selectivity of the reaction is 100%.

The tetrabutyl ammonium nitrate is not a product commercially easily available. It can be obtained from tetrabutyl ammonium bromide or chloride, products of ready commercial availability, by synthesizing the tetrabutyl ammonium hydroxide by an electrochemical process described by J.R. Ochoa and M. Tarancon in the Spanish patent 9001049 and in J. Applied Electrochem 21, 365 (1991) and later neutralizing with nitric acid after removing water by evaporating and crystallizing, there is obtained tetrabutyl ammonium nitrate in solid white at the point of fusion 110-120°C.

The following examples illustrate the invention, without limitations of the same.

Examples 1 and 2 illustrate the synthesis of p-toluene sulfonate of glycidil, precursor of glycidil nitrate.

EXAMPLE 1

Into the reaction container are introduced 20 g. glycidil (0.267 moles) (purified by distillation in vacuo at 140°C and 20 mmHg) and 100 ml pyridine. They are frozen at 50°C and 58.5 g. (0.305 moles) of p-toluenesulfonile chloride are slowly added while the temperature does not exceed 10°C. The final addition lasts 30 minutes, after which the mass of reaction is left to react at ambient temperature for 2 h. Subsequently, the mass of reaction is diluted with 150 ml $CH_2Cl_2$ and several times extracted with aqueous 15% HCl. The organic phase is dried with $MgSO_4$ filtered and $CH_2Cl_2$ is recovered by distillation in vacuo at 30°C. 51.5 g. of an oily product are obtained consisting of p-toluene sulfonate of glycidil (85%) and 50% not indentified polymer.

EXAMPLE 2

In a reactor of 5 1. are mixed 929.6 g. p-toluene sulfonile chloride (4.87 moles), 536 g. triethylamine (5.3 moles) and 3.7 kg. $CCl_4$. For one hour and under vigorous agitation are added 463 g. (6.25 moles) purified glycidil, taking care that the temperature does not exceed 30°C. Subsequently, the reaction is maintained at 25°C for 6 h. In the course of the reaction, there appears a white precipitate of triethylamine chlorhydrate.

After the reaction is finished, the salts are extracted 5 times at 40°C with 1800 $cm^3$ water. The organic phase is dried with anhydrous $Na_2SO_4$, filtered and $CCl_4$ is recovered by distillation in vacuo at 50°C.

The residue, 1000.2 g. is p-toluene sulfonate of glycidil which is crystallized when frozen. The fusion point (34-36°C). Purity NMR exceeds 98%. Yield 90.5%.

Spectroscopic data of the product:

$^1$H RMN: $\delta$ ($CDCl_3$) 2,5-2,9 (2H,m);3,20(1H,m);3,90-4,30(2H,m);7,80(2H,d);7,36(2H,d);2,46(3H,S) ppm

$^{13}$C RMN:$\delta$ ($CDCl_3$): 44,6; 48,9; 70,5; 132,6; 128,0; 130; 145,2; 21,7 ppm

IR: $\nu$ max (film liquido): 1595(s); 1491(w); 1357(s); 1189(s), 1175(s); 1095(s); 962 (s, breadth); 914(5) 829 (s, breadth), various overtapping bands); 664(s) $cm^{-1}$.

Examples 3-12 illustrate the synthesis of glycidil nitrate GN (III).

EXAMPLE 3

In a reaction container are mixed 10 g. p-toluene sulfonate of glycidil, 5.6 g. sodium nitrate and 30 g. DMA. After 5 h. of reaction, the yield is 95% (HPLC). 100 ml $CH_2Cl_2$ are added and extracted with 3x400 $cm^3$ water. The organic phase is dried with $MgSO_4$, filtered and dichloromethane is recovered by distillation at 30°C and 100 mmHg. The residual is 3.6 g. NMR indicates that it is formed by a mixture of glycidil nitrate (85%) and DMA (15%). The isolated yield is 58%.

The example illustrates the difficulty in separating glycidil nitrate of DMA and the entailed yield losses.

EXAMPLE 4

The process described in example 3 is repeated but with acetonitrile as solvent. The reaction temperature is that of the reflux. The yield is 0%.

EXAMPLE 5

In a reaction container are mixed 20G (0.087 moles) of p-toluene sulfonate of glycidil, 11 g. $NaNO_3$ (0.13 moles), 3.5 g. (0.0087 moles) p-toluene sulfonate of tetrabutyl ammonium and 20 g. of $CH_2Cl_2$. The reaction

is maintained at 40°C for 71 h. The yield of glycidil nitrate (GN) is 25%. The selectivity is 100%.

EXAMPLE 6

The process of example 5 is repeated but using $CHCl_3$ as solvent and operating at 60°C temperature. The reaction period was 71 h. The yield 50%. Selectivity is 100%.

EXAMPLE 7

The process of example 5 was repeated but using tetrafluoroborate of tetrabutyl ammonium with phase transfer catalyst. The yield 3%.

EXAMPLE 8

In a reactor of 2 1. are mixed 419.5 g. p-toluene sulfonate of glycidil (1.84 moles), 235 g. $NaNO_3$ (2.76 moles), 56.1 g. tetrabutyl ammoniun nitrate (0.18 moles) and 420 g. acetonitrile. The mixture is maintained in agitation for 6 h. at 85°C. After the reaction is finished, the solid is filtered and from the filtrate is recovered acetonitrile by distillation at 100 mmHg and 50°C. The residue in the boiler is distilled at 100°C and 1-3 mmHg obtaining 73.9 g. glycidil nitrate of purity above 99% as indicated by NMR and IR. This example, in comparison with Example 4, shows the effectiveness of PTC.

The residue in the boiler (316.4 g) is formed by the substrate without reaction, glycidil nitrate without reaction and quaternary ammonium salt. The yield of the reaction before separation was 58%. Once separated, the yield was 33.7%. The selectivity 100%.

The following examples show that the residual in the boiler can be recirculated many times without putting in again the quaternary ammonium salt.

EXAMPLE 9

To the residue in the boiler of example 8 are added 118 g. of $NO_3Na$ and 200 g. acetonitrile. The reaction is maintained 10 h. at 85°C. Subsequently, as in example 8, 53.8 g are distilled (pure glycidil nitrate) and the residue of 212 g. is obtained in the boiler formed by the substrate without reaction, glycidil nitrate and quaternary ammonium salt.

The yield of the reaction was 72%. The isolated output of the combined reactions described in this example and in the previous one was 58%. The selectivity was 100%.

EXAMPLE 10

300 g. glycidil p-toluenesulfate (1.315 moles), 350 g. $NO_3Na$ (4.125 moles) and 375 g. recovered acrylonitrile are added to the residue in the boiler of the previous example. The reaction was maintained at 85°C for 6 h. proceeding as in example 8. 135.6 g. of pure glycidil nitrate were isolated. The remainder in the boiler was 206 g. The yield before the isolation was 80%. The yield after isolation was 69%. Selectivity was 100%.

EXAMPLE 11

250 g. glycidil p-toluenesulfonate (1.09 moles) were added to the residue in the boiler of example 10. Also 363 g. of sodium nitrate (4.278 moles) and 300 g. recovered acetonitrile. After a reaction for 6 h. at 85°C, the procedure continued as in example 8. 120.6 g. pure glycidil nitrate were separated leaving in the boiler a residue of 178 g. formed by quaternary ammonium salt and the remainder predominantly by glycidil nitrate and some of the substrate without reaction.

The yield of the reaction was 95% and the selectivity 100%.

EXAMPLE 12

250 g. glycidol p-toluene sulfate (1.09 moles), 280 g. sodium nitrate (3.29 moles) and 300 g. acetonitrile were added to the residue in the boiler of Example 11. After reaction was maintained for 6 h. at 85°C the procedure followed as in Example 8. 95 g. pure glycidil nitrate were separated leaving in the boiler a residue formed by quaternary ammonium salt, glycidil nitrate and substrate without reaction. Its weight was 207.8 g. The yield of the reaction before separation was 80% and selectivity 100%.

Nothing indicated that in examples 8-12 the quaternary ammonium salt would be deactivated. The residue in the boiler could follow by recycling. The drop in the yield in example 12 relative to 11 was due to the smaller amount there of added $NO_3Na$.
Spectral data of glycidil nitrate:

$^1H$ RMN: δ ($CDCl_3$): 2,72 (1H,d,d); 2,92 (1H,t); 3,29 (1H,m); 4,77 (1H,d,d); 4,34 (1H,d,d) ppm

$^{13}C$ RMN: δ($CDCl_3$): 44,6; 47,7; 73,1 ppm

IR: ν max (liquid film ): 1632, 1424, 1343, 1280, 1133, 997, 968, 908, 858, 756, 663 $cm^{-1}$.

Examples 13-17 illustrate the synthesis of hydroxy-terminated GAP-PGN copolymers starting with PGN. The hydroxyl indices were determined by FTIR (Fourier Transformed Infrared Spectroscopy), molecular weights by GPC (Gel Permeation Chromatography) (THF, master agent polyethylene glycol) and the composition by $^1H$-NMR and elemental analysis.

EXAMPLE 13

Starting with PGN having the following characteristics:
Mw= 2400 (molecular weight average by weight)
Mn= 1680 (molecular weight average by number)
OH= 0.91 meq/g.
10 g. of said polymer dissolved in 30 g. DMA were reacted at 95°C with 8.2 g. sodium azide during 0.5 h., after which the mass of reaction was rapidly cooled, the precipitate filtrate and washed with DMA. It is recovered from the filtrate by distillation in vacuo. The residue in the boiler is dissolved in 100 ml $CH_2Cl_2$ and 3 times extracted with 300 ml water. The organic phase is dried with $MgSO_4$, and filtered, recovering dicholoromethane by distillation in vacuo. The residue consists of 9.2 g. oily liquid whose infrared spectrum and $^1H$-NMR indicates that it is a GAP-PGN copolymer with a 20% GAP content and 80% PGN. The obtained yield was 95.2%. Characteristics of the obtained GAP-PGN copolymer:
Mw= 2100, Mn= 1500, OH= 1.02 meq/g.

EXAMPLES 14-17

The process described in example 13 is repeated but with the following periods of reaction: 1 h., 2 h., 4 h., 6 h.
The results are shown in the following table:

| EXAMPLE | tr (h) | COMPOSITION | | $OH^-$ meq/g | Mw | Mn |
|---|---|---|---|---|---|---|
| | | GAP% - | PGN% | | | |
| 14 | 1 | 35 | 65 | 1,06 | 2020 | 1390 |
| 15 | 2 | 60 | 40 | 1,10 | 1950 | 1340 |
| 16 | 4 | 80,5 | 19,5 | 1,16 | 1880 | 1260 |
| 17 | 6 | 93 | 7 | 1,20 | 1830 | 1220 |

The yield was between 90 and 95% in all cases.
Spectral data of GAP-PGN copolymers:
The intensity of the bands depends on the composition of the copolymer.
'H RMN: ($CDCl_3$):4,4-4,65 (2H,m); 3,40-3,70 (3H,m); 3,25-3,75 (2H,m) ppm
The first signal corresponds to the methylene joined to the nitrate group, the second to protons of the macromolecular chain of ethylene polyoxide and the last one to methylene joined to the azide group.
FTIR: ν max ( liquid film ): 3400, 2950, 2100, 1636, 1450, 1400, 1282, 1120, 865 $cm^{-1}$.
Examples 18 to 20 illustrate the synthesis of GAP by FOurier Transformed Infrared Spectroscopy (FTIR), NMR, Gel Permeation Chromatography (GPC), (THF, polyethylene glycol as master agent) and elemental analysis.

EXAMPLE 18

One starts with polyglycidil nitrate having the following characteristics:
Mw= 2400

Mn= 1680
OH= 0.91 meq/g
10 g. of said polymer, dissolved in 30 g. dimethyl acetamide (DMA), are reacted at 95°C with 8.2 g. sodium azide. After 10 h. of reaction, the precipitate is filtered and washed with DMA. The DMA of the filtrate is recovered by distillation. The residue in the boiler is dissolved in 100 ml $CH_2Cl_2$ and is 3 times extracted with 300 ml $H_2O$. The organic phase is dried with $Na_2SO_4$, and filtered, recovering the dichloromethane by distillation. The residue is constituted by 7.65 g. a reddish viscous liquid whose infrared spectrum and nuclear magnetic resonance indicate that it is GAP.
Mw= 1900
Mn= 1300
OH= 1.15 meq/g
NMR determined that the content of azide groups was 98% and of the residual nitrate groups 2%. Elemental analysis:
C: 35.8%
H: 5.12%
N: 41.7%

EXAMPLE 19

One starts with PGN having the following characteristics
Mw= 2130
Mn= 1420
OH= 0.97 meq/g
10 g. of said polymer, dissolved in 25 g. DMF are reacted with 8.2 g sodium azide. After 24 h.reaction the precipitate is filtered and washed with DMA. The DMA is recovered by distillation. DMF is recovered by distillation. The polymer is isolated by the process described in Example 18. 7 g. of a viscous reddish liquid are obtained whose spectra IR and MMR indicate that it is GAP with a substitution degree of 99%.
Mw= 1900
Mn= 1300
OH= 1.15 meq/g
It was determined for NMR that the content of azide groups was 98% and of residual nitrate groups 2%.
Characteristics of obtained GAP:
Mw= 1743
Mn= 1020
OH= 1.50 meq/g
Elemental analysis:
C: 35.8%
H: 5.12%
N: 41.7%EXAMPLE 20

Example 20

The process described in example 1 was repeated, but adding 3.84 g. tetrabutyl ammonium nitrate. The characteristics of the starting PGN were as follows:
Mw= 2140
Mn= 1390
OH= 0.97 meq/g
A GAP was obtained with a 98% content of azide groups after 4.7 h. with the following characteristics:
Mw= 1690
Mn= 1050
OH= 1.47 meq/g
Having described the object of the present invention, the gist of the invention will now be defined in the following claims.

## Claims

1. A hydroxy-terminated glycidil azide-glycidil nitrate (GAP-PGN), characterized by being constituted by an

aliphatic polyether, with methylized and methylnitrated groups pending from its macromolecular chain, of the general formula (I)

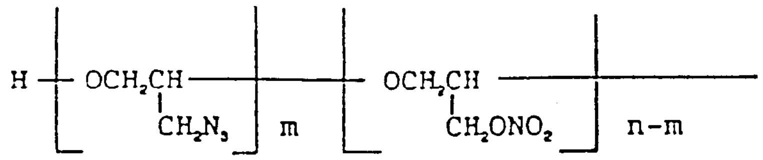

(I)

wherein n is a whole number between 8 and 84, and m is a whole number greater than zero and smaller than n.

2. The process for producing a GAP-PGN copolymer of the general formula (I)

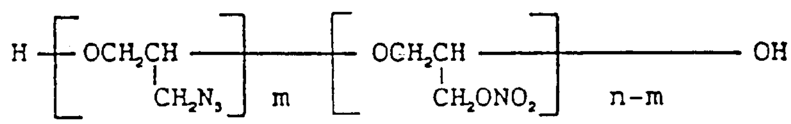

(I)

wherein n is a whole number between 8 and 84, and m is a whole number greater than zero and smaller than n. characterized by reacting a hydroxy-terminated polyglycidil nitrate (PGN) of general formula (II)

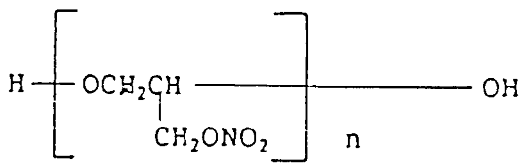

(II)

wherein n is a whole number between 8 and 84, with a metallic azide in a suitable organic solvent, optionally in the presence of a phase transfer catalyst (PTC).

3. The process according to claim 2, characterized in that the metallic azide is sodium azide.

4. The process according to claim 2, characterized in that as solvent can be used DMA, DMF, NMP, NMF, DMSO, ACN or sulfolane, preferably DMA, DMF or DMSO.

5. The process according to claim 2, characterized by being carried out at a temperature between 0 and 120°C, preferably between 50 and 100°C.

6. The process according to claim 2, characterized in that as CTF, can be used quaternary ammonium salts, preferably tetrabutyl ammonium p-toluene sulfonate or tetrabutyl ammonium nitrate.

7. The process according to claim 2, characterized in that the molar ratio azide/PGN is selected as a function of the intended composition of GAP-PGN copolymer to be obtained, preferably with a slight stoichiometric molar excess of azide.

8. A process for producing glycidil nitrate (GN) of the general formula (III)

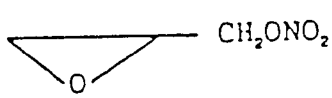

(III)

characterized by the following steps:

a) reaction at a temperature between ambient temperature and 50°C, of glycidol of the formula

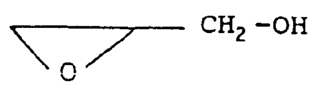

with sulfonic chloride acid of formula (V)

$R\text{-}SO_2\text{-}Cl$ (V)

wherein

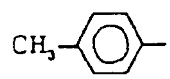 ;

$CH_3$-; $CH_3\text{-}CH_2$-; $CH_3(CH_2)_2$-; $CH_3(CH_2)_3$-;
in an inert solvent and in the presence of an organic basis.

b) separation of the obtained glycidol sulfonic ester of general formula (VI)

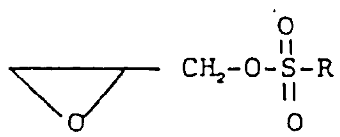 (VI)

wherein R stands for earlier mentioned significance.

c) reaction of sulfonic ester (VI) with a metallic nitrate in a suitable organic solvent, optionally in the presence of a phase transfer catalyst (PTC), at temperatures between ambient temperature and 100°C,and

d) separation the obtained glycidil nitrate (III) and the reuse of the solvent and of the phase transfer catalyst in case it has been used.

**9.** The process according to claim 8, wherein the solvent for synthesizing the sulfonic ester of glycidil can be pyridine or a chlorinated organic solvent, preferably $CCl_4$.

**10.** The process according to claim 8, wherein the bases can be any organic bases capable of rapidly reacting with hydrochloric acid and without interfering in the reaction, preferably pyridine or triethylamine.

**11.** The process according to claim 8, wherein the metallic nitrate is preferably sodium nitrate.

**12.** The process according to claim 8, wherein the reaction of the sulfonic ester with the metallic nitrate is carried out in a polar organic solvent such as DMA, DMF, NMF, NMP, DMSO, sulfolane, THF, dioxane, acetonitrile, dichloromethane, chloroform, $CCl_4$ and 1.2 dichloroethane.

**13.** The process according to claim 8, wherein the phase transfer catalyst, in case of being used, is a quaternary ammonium salt, preferably tetrabutyl ammonium p-toluene sulfonate or tetrabutyl ammonium nitrate.

**14.** The process according to claim 8, wherein the preferred solvent is acetonitrile and the phase transfer catalyst is tetrabutyl ammonium nitrate.

**15.** The process according to claim 8, wherein the solvent and the phase transfer catalyst are recycled many times, normally at least 15 times and at most 25 times.

**16.** The process according to claim 8, wherein the molar ratio of metallic nitrate/sulfonic ester (VI) is between 1 and 3.

**17.** The process according to claim 8, wherein the molar ratio CTF (if used)/sulfonic ester (VI) is between 0.05 and 0.20, preferably between 0.1 and 0.15.

**18.** A process for producing a hydroxy-terminated azide glycidil polymer (GAP) of functionality equal to or more than 2 and of general formula (VII)

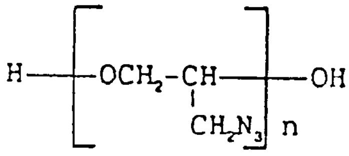 (VII)

wherein n is a whole number between 8 and 84. characterized by reacting hydroxy-terminated polyglycidin nitrate (PGN), of the general formula (II)

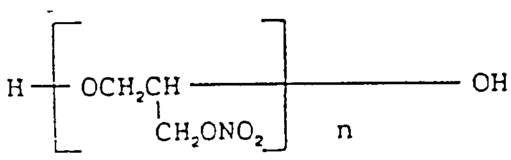 II

wherein n is a whole number between 8 and 84, with metallic azide, in a polar organic solvent, optionally in the presence of a phase transfer catalyst.

**19.** The process according to claim 18, characterized in that the polar organic solvent is preferably selected from the group of dimethyl acetamide, dimethyl formamide, N-methyl pyrolidon, dimethyl sulfoxide and sulfolane.

**20.** The process according to claim 18, characterized in that metallic azide is sodium azide.

**21.** The process according to claim 18, characterized in that the molecular weight of PGN is between 1200 and 10000.

**22.** The process according to claim 18, characterized in that the reaction temperature is between 50 and 120°C, preferably between 90 and 105°C.

**23.** The process according to claim 18, wherein, when the phase transfer catalyst is used, it can be any quaternary ammonium salt, preferably tetrabutyl ammonium p-toluene sulfonate and tetrabutyl ammonium nitrate.

**24.** The process according to claims 18 and 20, characterized in that the molar ratio of metallic azide and PGN is between 1 and 2, preferably 1.5.

European Patent Office

# EUROPEAN SEARCH REPORT

Application Number
EP 93 50 0116

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | EP-A-0 485 099 (THIOKOL CORPORATION) * page 5, line 40 - page 6, line 45 * --- | 8-10,17 | C08G65/32 C08G65/22 C07D303/16 C06B45/10 |
| A | EP-A-0 293 555 (ROCKWELL INTERNATIONAL CORP.) * claim 1 * --- | 1,8,18 | |
| A | US-H-H272 (GILBERT E.E.) * claim 1; example 1 * ----- | 1 | |

TECHNICAL FIELDS SEARCHED (Int.Cl.5)
C08G
C07D
C06B

The present search report has been drawn up for all claims

| Place of search | Date of completion of the search | Examiner |
|---|---|---|
| THE HAGUE | 4 February 1994 | O'Sullivan, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)